# EUROPEAN PATENT APPLICATION

(11) **EP 3 076 163 A1**
(43) Date of publication of application: **05.10.2016**
(21) Application number: 14865249.8
(22) Date of filing: 17.11.2014
(51) Int. Cl.: G01N 21/892, B41F 33/14

(54) **INSPECTION DEVICE FOR SHEET-SHAPED OBJECT**

(30) Priority: 29.11.2013 JP 2013247012
(71) Applicant: Komori Corporation, Sumida-ku Tokyo 130-0001 (JP)
(72) Inventor: OHAMA, Kentaro, Tsukuba-shi Ibaraki 300-1268 (JP)
(74) Representative: Uexküll & Stolberg
(86) International application number: PCT/JP2014/080299
(87) International publication number: WO 2015/079937

(57) **Abstract**

An inspection device (40) is configured to be provided with: white LED illuminators (41a1, 41a2, 41b1, 41b2); IR-LED illuminators (43a, 43b); visible range color cameras (42A, 42B) ; an IR monochrome camera (44) which is provided at a position outside the fields of view of the cameras (42A, 42B); support frames (49a, 49b) which support the illuminators (41a1, 41a2, 41b1, 41b2, 43a, 43b); an IR filter (45) which causes only infrared rays to be incident on the camera (44); and a control unit (46) which, according to visible light incident on the cameras (42A, 42B), determines whether a pattern portion printed on paper (W) by ordinary ink is appropriate or not, and according to the infrared rays incident on the camera (44), determines whether a portion in which a "security thread" is processed and a portion in which an "OVD" is processed of the paper (W) are appropriate or not.

## Description

### Technical Field

The present invention relates to an inspection device for a sheet including a pattern portion printed by using an ordinary ink and a portion processed by using mirror-surfaced members such as a security thread and an OVD (Optical Variable Device).

### Background Art

Printed products such as bank-notes and securities are often processed, on and inside their paper surfaces, by using a "security thread, "an "OVD," and the like to be mirror-surfaced for counterfeit prevention. Note that the "security thread" is a strip-shaped object made of plastic or the like and sewed or weaved in a bank-note sheet or the like to be visible when held against light. Meanwhile the "OVD" is an iridescent object made of a hologram, a foil, and the like and is embedded in the bank-note sheet or the like to exhibit various tilt effects (to exhibit such effects that an image moves and colors change when tilted). Both of the "security thread" and the "OVD" are mirror-surfaced.

Accordingly, in addition to a pattern portion printed by using an ordinary visible ink and the like, a portion processed by using the "security thread," the "OVD," and the like needs to be inspected with an effect of the color change therein reduced to minimum.

### Citation List

### Patent Literature

Patent Literature 1: Japanese Patent Application Publication No. 2010-221410

### Summary of Invention

### Technical Problem

Although various inspection devices for the pattern portion printed by using the ordinary visible ink have been conventionally proposed as in, for example, Patent Literature 1, there has been no means for accurately detecting the position, the shape, and the like of the portion processed by using the "security thread," the "OVD," and the like.

In view of this, an object of the present invention is to provide an inspection device for a sheet which can inspect in detail a pattern portion printed by using an ordinary ink and a portion processed by using a mirror-surfaced member.

### Solution to Problem

An inspection device for a sheet of the present invention for solving the problems described above is an inspection device for a sheet including a pattern portion printed by using an ordinary ink and a portion processed by using a mirror-surfaced member, characterized in that the inspection device comprises: a white light source configured to emit a light including visible light onto the sheet; an infrared light source configured to emit a light including infrared light onto the sheet; two visible light imaging means for imaging the sheet; one infrared light imaging means for imaging the sheet, the infrared imaging means provided at such a position that the infrared light imaging means is outside fields of view of the two visible light imaging means; an infrared light filter provided to filter out the visible light in the lights from the white light source and the infrared light source which is reflected on the sheet and allow only the infrared light to be incident on the infrared light imaging means; control means for determining appropriateness of the pattern portion printed on the sheet by using the ordinary ink, by means of the visible light from the white light source and the infrared light source which is reflected on the sheet to be incident on the two visible light imaging means and for determining appropriateness of the portion processed by using the mirror-surfaced member in the sheet, by means of the infrared light from the white light source and the infrared light source which is reflected on the sheet to be incident on the one infrared light imaging means.

Moreover, the inspection device for a sheet of the present invention is characterized in that, in the aforementioned inspection device for a sheet, the one infrared light imaging means is provided between the two visible light imaging means provided away from each other in a width direction of the sheet.

Furthermore, the inspection device for a sheet of the present invention is characterized in that the aforementioned inspection device for a sheet further comprises an imaging means supporting member supporting all of the two visible light imaging means and the one infrared light imaging means.

Moreover, the inspection device for a sheet of the present invention is characterized in that, in the aforementioned inspection device for a sheet, the two visible light imaging means and the one infrared light imaging means are provided at positions away from an imaged surface of the sheet by an equal distance.

Furthermore, the inspection device for a sheet of the present invention is characterized in that the aforementioned inspection device for a sheet further comprises a light source supporting member which supports both of the white light source and the infrared light source such that the light from the white light source and the light from the infrared light source fall on an imaged surface of the sheet imaged by the two visible light imaging means and the one infrared light imaging means and in which a supporting surface supporting the infrared light source is inclined with respect to a supporting surface supporting the white light source such that an angle formed by the imaged surface of the sheet and a direction of the light emitted by the infrared light source is smaller than an angle formed by the imaged surface of the sheet and a direction of the light emitted by the white light source.

### Advantageous Effects of Invention

In the inspection device for a sheet of the present invention, specular reflection of the lights emitted from the light sources occurs on surfaces of the mirror-surfaced member, and the visible light is entirely filtered out by the infrared light filter. The mirror-surfaced member on which the specular reflection occurs thus appears black to the imaging means. Meanwhile, in the normal pattern portion, diffuse reflection of the infrared light occurs, and the infrared light is incident on the imaging means as it is. The pattern portion thus appears white to the imaging means. Hence, the portion of the sheet processed by using the mirror-surfaced member can be inspected in detail, i.e. can be clearly distinguished from the normal pattern portion.

### Brief Description of Drawings

[Fig. 1] Fig. 1 is a schematic configuration diagram of a main embodiment of an inspection device for a sheet of the present invention.
[Fig. 2] Fig. 2 is a view in a direction of the arrow II in Fig. 1.
[Fig. 3] Fig. 3 is a control block diagram of the inspection device in Fig. 1.
[Fig. 4] Fig. 4 is an explanatory view of a pattern in a small piece of paper.
[Fig. 5] Fig. 5 is an explanatory view of another pattern in a small piece of paper.

### Description of Embodiments

An embodiment of an inspection device for a sheet of the present invention is described based on the drawings. However, the present invention is not limited to the embodiment described below based on the drawings.

### <Main Embodiment>

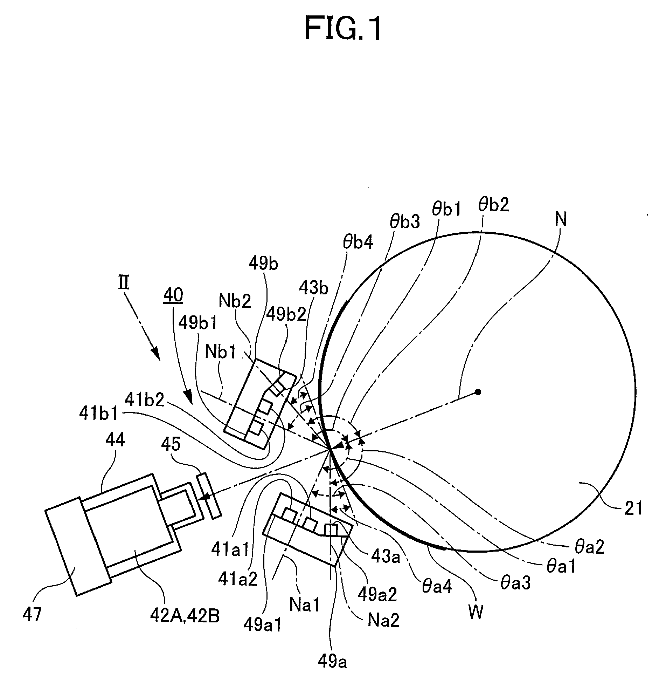

A main embodiment of the inspection device for a sheet of the present invention is described based on Figs. 1 to 5.

As illustrated in Fig. 1, paper W which is a sheet including a pattern portion printed by using an ordinary ink and a portion processed by using a mirror-surfaced member such as a "security thread" and an "OVD" is transferred from a paper feeding tray of a paper feeding unit to an inspection cylinder 21 which is a holding cylinder, and print quality of the paper W is inspected by an inspection device 40 provided to face a peripheral surface of the inspection cylinder 21. Then, the paper W is sent to a paper delivery unit via not-illustrated conveying means and is stacked on a paper delivery tray.

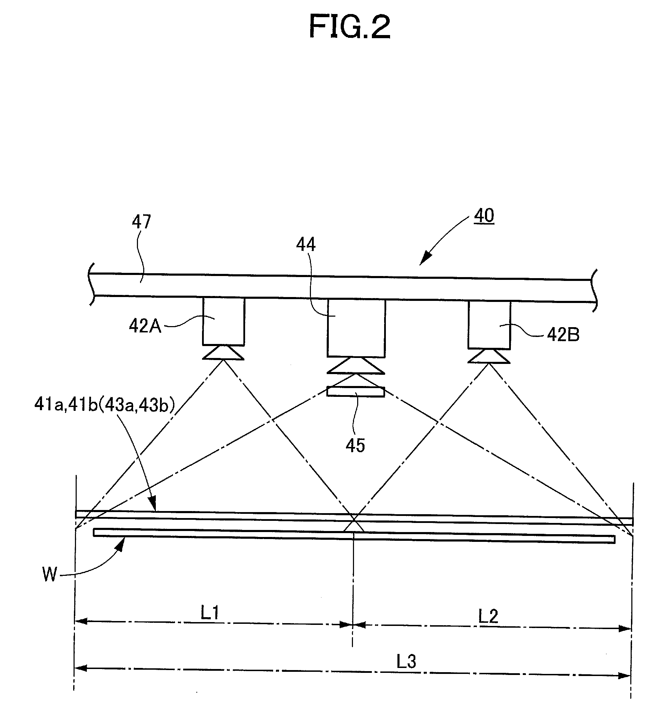

As illustrated in Figs. 1 and 2, the inspection device 40 includes, as devices for visible ink inspection: two sets of paired white LED illuminators 41a1, 41a2 and paired white LED illuminators 41b1, 41b2 which are white light sources configured to emit a light including visible light on the paper W; and two visible color cameras 42A, 42B which are visible light imaging means for imaging the paper W.

In addition, the inspection device 40 includes, as devices for inspection of the portion of the paper W processed by using the "security thread" and the "OVD" : one set of paired IR-LED illuminators 43a, 43b which are infrared light sources configured to emit a light including infrared on the paper W; one IR monochrome camera 44 which is infrared light imaging means for imaging the paper W; and an infrared filter (IR filter) 45 which filters out light with a wavelength equal to or shorter than the visible light in the lights from the white LED illuminators 41a1, 41a2, 41b1, 41b2 and the IR-LED illuminators 43a, 43b reflected on the paper W and allows only infrared light to be incident the IR monochrome camera 44.

The one IR monochrome camera 44 is provided in the middle of the two visible color cameras 42A, 42B provided away from each other in a width direction of the paper W, and is installed at a position where the IR monochrome camera 44 does not enter the fields of view of the two visible color cameras 42A, 42B. In Fig. 2, L1 denotes an inspection width of the visible color camera 42A, L2 denotes an inspection width of the visible color camera 42B, and L3 denotes an inspection width of the IR monochrome camera 44.

Moreover, each of the two visible color cameras 42A, 42B and the one IR monochrome camera 44 is arranged such that an optical axis thereof is aligned with a normal line N to an outer peripheral surface of the inspection cylinder 21, and images an imaged surface of the paper W in a direction perpendicular thereto. Moreover, the visible color cameras 42A, 42B and the IR monochrome camera 44 are provided at positions away from the imaged surface of the paper W by an equal distance. Specifically, the three cameras 42A, 42B, 44 are all supported on the same supporting frame 47 which is an imaging means supporting member whose longitudinal direction extends in an axial direction of the inspection cylinder 21.

The white LED illuminators 41a1, 41a2 and the IR LED illuminator 43a are all supported on the same supporting frame 49a whose longitudinal direction extends in the axial direction of the inspection cylinder 21. The white LED illuminators 41b1, 41b2 and the IR LED illuminator 43b are all supported on the same supporting frame 49b whose longitudinal direction extends in the axial direction of the inspection cylinder 21.

The supporting frames 49a, 49b support the white LED illuminators 41a1, 41a2, 41b1, 41b2 and the IR-LED illuminators 43a, 43b such that the light including visible light from the white LED illuminators 41a1, 41a2, 41b1, 41b2 and the light including infrared light from the IR-LED illuminators 43a, 43b fall on the imaged surface of the paper W on the inspection cylinder 21 in directions inclined with respect to the normal line N, the imaged surface being imaged by the two visible color cameras 42A, 42B and the one IR monochrome camera 44. Moreover, supporting surfaces 49a2, 49b2 which support the IR-LED illuminators 43a, 43b are inclined with respect to supporting surfaces 49a1, 49b1 which support the white LED illuminators 41a1, 41a2, 41b1, 41b2 such that angles θa2, θb2 formed by the normal line N and perpendicular lines Na2, Nb2 perpendicular to the supporting surfaces 49a2, 49b2 are smaller than angles θa1, θb1 formed by the normal line N and perpendicular lines Na1, Nb1 perpendicular to the supporting surfaces 49a1, 49b1, i.e. such that angles θa4, θb4 formed by the imaged surface of the paper W and directions of the lights emitted by the IR-LED illuminators 43a, 43b are smaller than angles θa3, θb3 formed by the imaged surface of the paper W and directions of the lights emitted by the white LED illuminators 41a1, 41a2, 41b1, 41b2. In the embodiment, a light source supporting member includes the supporting frames 49a, 49b as described above.

The inspection device 40 also includes a control device 46 which is control means for: determining appropriateness of the pattern portion of the paper W printed by using the ordinary ink (hereafter, referred to as "visible ink"), by means of the visible light from the white LED illuminators 41a1, 41a2, 41b1, 41b2 and the IR-LED illuminators 43a, 43b which is reflected on the paper W to be incident on the two visible color cameras 42A, 42B; and determining appropriateness of the shape of the portion of the paper W processed by using the "security thread" and the "OVD," by means of the infrared light from the white LED illuminators 41a1, 41a2, 41b1, 41b2 and the IR-LED illuminators 43a, 43b which is reflected on the paper W to be incident on the one IR monochrome camera 44.

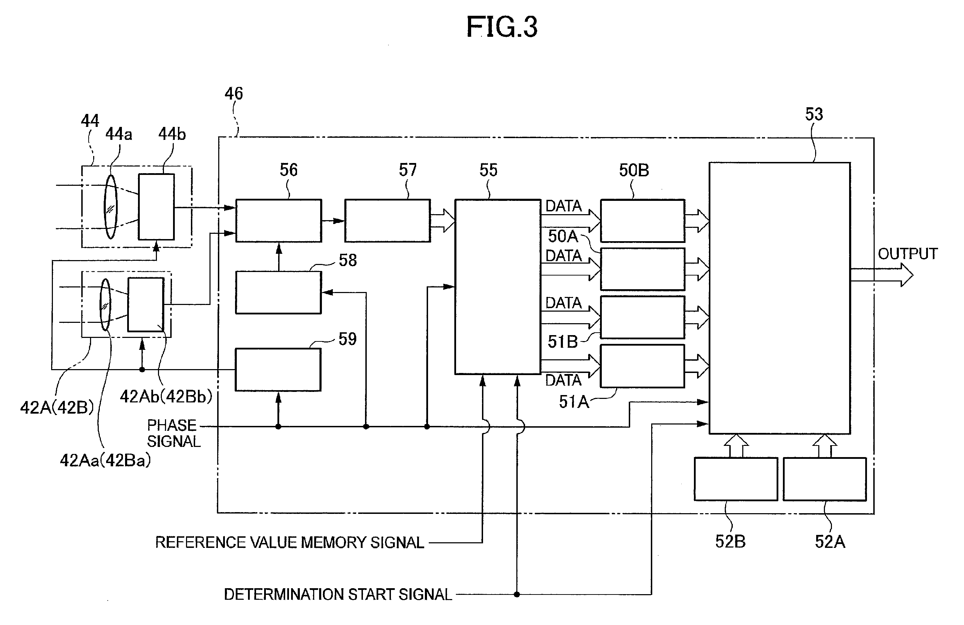

Optical and electrical configurations of the inspection device 40 are described based on Fig. 3. The inspection device 40 includes: the aforementioned two visible color cameras 42A, 42B configured to image the pattern portion of the paper W printed by using the visible ink; the aforementioned one IR monochrome camera 44 configured to image the portion of the paper W processed by using the "security thread" and the "OVD"; and the control device 46 configured to inspect the print quality of patterns printed on the paper W based on imaging output from the cameras 42A, 42B, 44. The cameras 42A, 42B, 44 include: optical systems including lenses 42Aa, 42Ba, 44a; and CCDs (Charged Coupled Devices) 42Ab, 42Bb, 44b configured to convert images focused by the optical systems into electric signals.

The control device 46 includes: reference memories 50A (for the IR monochrome camera), 50B (for the visible color cameras) which store reference image signals; detection memories 51A (for the IR monochrome camera), 51B (for the visible color cameras) which store detection image signals; a memory controller 55 which controls reading from and writing to the reference memories 50A, 50B and the detection memories 51A, 51B; determination level setting circuits 52A (for the IR monochrome camera), 52B (for the visible color cameras) in each of which an allowable level difference of two signals read from a pair of the reference memory 50A and the detection memory 51A or a pair of the reference memory 50B and the detection memory 51B is set; a comparison circuit 53 which compares the two signals in consideration of the allowable level difference set in the determination level setting circuit 52A, 52B; an amplifier 56 which amplifies output of the CCDs 42Ab, 42Bb, 44b; an A/D convertor 57 which performs analog/digital conversion on output of the amplifier 56 and outputs the converted output to the memory controller 55; a correction circuit 58 which adjusts a gain of the amplifier 56; and a CCD controller 59 which controls the CCDs 42Ab, 42Bb, 44b.

The reference memories 50A, 50B store reference image data read from the pattern portion of the paper W normally printed by using the visible ink and from the portion of the paper W normally processed by using the "security thread" and the "OVD," at a start of a print job. Note that the reference image data stored in the reference memories 50A, 50B varies by print job. The detection memories 51A, 51B store detection image data read from the paper W which is an inspection target.

The memory controller 55 controls reading and writing of data from and to the reference memories 50A, 50B and the detection memories 51A, 51B. In each of the determination level setting circuits 52A (for the IR monochrome camera), 52B (for the visible color camera), there is set the allowable level difference between the reference image data read from the reference memory 50A, 50B and the detection image data read from the detection memory 51A, 51B.

The comparison circuit 53 outputs a signal indicating that the quality is not good when the level difference between the reference image data and the detection image data which are read by the memory controller 55 is equal to or greater than the allowable level difference set in the determination level setting circuit 52A, 52B. Specifically, the comparison circuit 53 compares the reference image data and the detection image data which correspond to each of pixels of the CCDs 42Ab, 42Bb, 44b to compare the levels of the reference image data and the detection image data which correspond to each pixel, and outputs the no-good signal when the level difference is equal to or greater than the allowable level difference in any one of the pixels.

To be more specific, the comparison circuit 53 performs a first comparison operation of comparing the reference image data read from the reference memory 50A, 50B with the detection image data read from the detection memory 51A, 51B sequentially for each of the pixels. Next, the comparison circuit 53 performs a second comparison operation of comparing the level difference between the two signals obtained in the first comparison operation with the allowable level difference outputted from the determination level setting circuit 52A, 52B. When the level difference between the two signals is greater than the allowable level difference in a result of the second comparison operation, the comparison circuit 53 outputs the no-good signal which indicates that the pattern portion of the paper W printed by using the visible ink or the portion of the paper W processed by using the "security thread" and the "OVD" is not good, the paper W being the inspection target.

The correction circuit 58 adjusts the gain of the amplifier 56 according to a rotation speed of the inspection cylinder 21. Specifically, since the output levels of the CCDs 42Ab, 42Bb, 44b decrease as the rotation speed of the inspection cylinder 21 increases even when the amount of light incident on each of the cameras 42A, 42B, 44 are the same, the correction circuit 58 eliminates effects of the rotation speed. A phase signal is supplied to the memory controller 55, the comparison circuit 53, the correction circuit 58, and the CCD controller 59. A reference value memory signal is supplied to the memory controller 55. A determination start signal is supplied to the memory controller 55 and the comparison circuit 53.

The phase signal is generated from an output signal of a not-illustrated rotary encoder which detects a rotation phase of the inspection cylinder 21. The phase signal includes a reference pulse which rises every time the inspection cylinder 21 rotates once and a clock pulse which rises every time the inspection cylinder 21 rotates a certain number of times. The reference value memory signal is a signal for reading the reference image data into the reference memories 50A, 50B via the memory controller 55 and is supplied by an operation on a reference value memory switch (not illustrated) performed by an operator. The determination start signal is a signal instructing a start of the comparison operation of the reference image data and the detection image data and is supplied by an operation on a determination start switch (not illustrated) performed by the operator.

The inspection device 40 configured as described above first performs, as pre-processing of a quality inspection step, capturing of the reference image data for each of the pattern portion of the paper W normally printed by using the visible ink and the portion of the paper W normally processed by using the "security threads" and the "OVD." Specifically, the operator checks a print state of the paper W in test printing using the paper W. When the operator determines that the print state is good, the operator operates the reference value memory switch (not illustrated) to start the supply of the reference value memory signal to the control device 46. When a reference signal indicating a reference position of the inspection cylinder 21 is outputted from the rotary encoder, capturing of the reference image data from the paper W conveyed while being held by the inspection cylinder 21 is started, and the reference image data is stored in the reference memories 50A, 50B.

After the reference image data of the paper W conveyed while being held by the inspection cylinder 21 is stored in the reference memories 50A, 50B, the determination processing is started in response to the determination start signal. In the determination processing, the detection image data of the paper W conveyed while being held by the inspection cylinder 21 for each of the detection memories 51A, 51B is read with the rotation of the inspection cylinder 21, as in the reading of the reference image data. Next, the detection image data read in the determination processing is compared with the reference image data stored in advance, and the appropriateness of the pattern portion of the paper W printed by using the visible ink and the appropriateness of the portion of the paper W processed by using the "security thread" and the "OVD" are determined depending on whether the difference between the level values of the reference image data and the detection image data is within the allowable level difference.

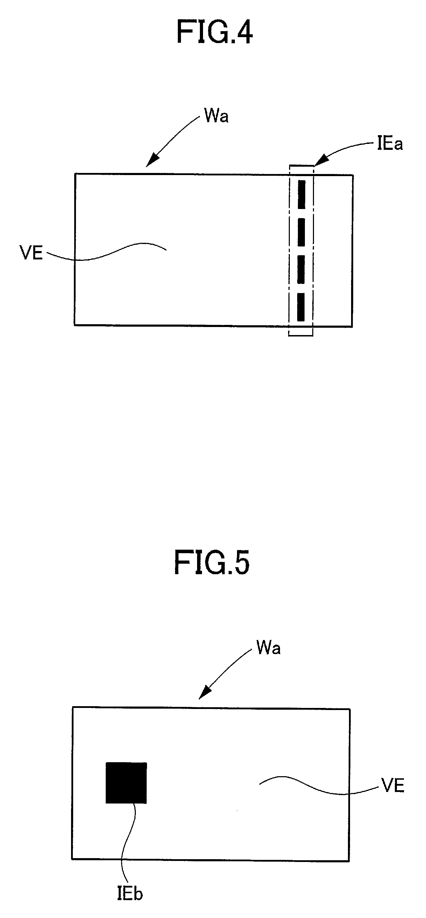

For example, as illustrated in Figs. 4 and 5, in a pattern portion VE printed by using the visible ink in each of many small pieces (bank-notes, securities, and the like) Wa of the paper W being the inspection target, the visible light emitted from the white LED illuminators 41a1, 41a2, 41b1, 41b2 and the IR-LED illuminators 43a, 43b to the imaged surface and reflected thereon is entirely filtered out by the IR filter 45 while the infrared light emitted to the imaged surface and reflected thereon is incident on the IR monochrome camera 44 as it is. Accordingly, the pattern portion VE appears entirely white to the IR monochrome camera 44.

Meanwhile, in a portion IEa (see Fig. 4) processed by using the "security thread" and a portion IEb (see Fig. 5) processed by using the "OVD," specular reflection of the light emitted from the white LED illuminators 41a1, 41a2, 41b1, 41b2 and the IR-LED illuminators 43a, 43b to the imaged surface occurs on the portion IEa processed by using the "security thread" and the portion processed by using the "OVD," and the visible light is entirely filtered out by the IR filter 45. Accordingly, no light including ambient light is incident on the IR monochrome camera 44, and the portion IEa processed by using the "security thread" and the portion IEb processed by using the "OVD" appear black to the IR monochrome camera 44.

As a result, detailed inspection of the positions and shapes of the portion IEa processed by using the "security thread" and the portion IEb processed by using the "OVD" is made possible. Moreover, since the visible color cameras 42A, 42B capture the colors of the pattern portion VE printed by using the visible ink as in the vision of human eyes, the detailed inspection of the pattern portion VE printed by using the visible ink is possible. Note that the number of windows can be also inspected in the portion IEa processed by using the "security thread."

As described above, since the visible light and the infrared light are different in wavelength, the pattern portion VE printed by using the visible ink, the portion IEa processed by using the "security thread", and the portion IEb processed by using the "OVD" can be inspected simultaneously on the same imaged surface.

As described above, in the embodiment, since the pattern portion printed by using the ordinary ink and the portion processed by using the mirror-surfaced member can be inspected in detail, the print quality of the paper W can be further improved.

Furthermore, the supporting frames 49a, 49b support the white LED illuminators 41a1, 41a2, 41b1, 41b2 and the IR-LED illuminators 43a, 43b such that the light including visible light from the white LED illuminators 41a1, 41a2, 41b1, 41b2 and the light including infrared light from the IR-LED illuminators 43a, 43b fall on the imaged surface of the paper W on the inspection cylinder 21 in the directions inclined with respect to the normal line N, the imaged surface being imaged by the two visible color cameras 42A, 42B and the one IR monochrome camera 44. Moreover, the supporting surfaces 49a2, 49b2 which support the IR-LED illuminators 43a, 43b are inclined with respect to the supporting surfaces 49a1, 49b1 which support the white LED illuminators 41a1, 41a2, 41b1, 41b2 such that the angles θa2, θb2 formed by the normal line N and the perpendicular lines Na2, Nb2 perpendicular to the supporting surfaces 49a2, 49b2 are smaller than the angles θa1, θb1 formed by the normal line N and the perpendicular lines Na1, Nb1 perpendicular to the supporting surfaces 49a1, 49b1, i.e. such that the angles θa4, θb4 formed by the imaged surface of the paper W and the directions of the lights emitted by the IR-LED illuminators 43a, 43b are smaller than the angles θa3, θb3 formed by the imaged surface of the paper W and the directions of the lights emitted by the white LED illuminators 41a1, 41a2, 41b1, 41b2. Accordingly, the directions of the white LED illuminators 41a1, 41a2, 41b1, 41b2 and the IR-LED illuminators 43a, 43b can be adjusted in one operation only by adjusting the directions of the supporting frames 49a, 49b, without causing deviation in the illuminating directions of the white LED illuminators 41a1, 41a2, 41b1, 41b2 and the IR-LED illuminators 43a, 43b. This can facilitate adjustment work performed by the operator.

Moreover, since the supporting frame 47 supports all of the two visible color cameras 42A, 42B and the one IR monochrome camera 44, the directions of the cameras 42A, 42B, 44 can be adjusted in one operation only by adjusting the direction of the supporting frame 47, without causing deviation in imaging directions of the two visible color cameras 42A, 42B and the one IR monochrome camera 44. This can facilitate the adjustment work performed by the operator.

Note that the present invention is not limited to the embodiment described above, and various changes can be made within a scope not departing from the spirit of the present invention.

### Industrial Applicability

The inspection device for a sheet of the present invention can inspect in detail the pattern portion printed by using the ordinary ink and the portion processed by using the mirror-surfaced member. Accordingly, the inspection device is very useful when used in printing industries of printing bank-notes and securities in which quality control is important.

### Reference Signs List

40 inspection device
41a1, 41a2, 41b1, 41b2 white LED illuminators
42A, 42B visible color camera
42Aa, 42Balens
42Ab, 42BbCCD
43a, 43b IR-LED illuminator
44 IR monochrome camera
44a lens
44b CCD
45 IR filter
46 control device
47 supporting frame
49a, 49b supporting frame
50A reference memory (for IR monochrome camera)
50B reference memory (for visible color cameras)
51A detection memory (for IR monochrome camera)
51B detection memory (for visible color cameras)
52A determination level setting circuit (for IR monochrome camera)
52B determination level setting circuit (for visible color cameras)
53 comparison circuit
55 memory controller
56 amplifier
57 A/D convertor
58 correction circuit
59 CCD controller
W paper
Wa small piece (bank-notes, securities, or the like)
VE pattern portion
IEa portion processed by using "security thread"
IEb portion processed by using "OVD"
L1 inspection width of visible color camera 42A
L2 inspection width of visible color camera 42B
L3 inspection width of IR monochrome camera 44

## Claims

1. An inspection device for a sheet including a pattern portion printed by using an ordinary ink and a portion processed by using a mirror-surfaced member, **characterized in that** the inspection device comprises:
a white light source configured to emit a light including visible light onto the sheet;
an infrared light source configured to emit a light including infrared light onto the sheet;
two visible light imaging means for imaging the sheet;
one infrared light imaging means for imaging the sheet, the infrared imaging means provided at such a position that the infrared light imaging means is outside fields of view of the two visible light imaging means;
an infrared light filter provided to filter out the visible light in the lights from the white light source and the infrared light source which is reflected on the sheet and allow only the infrared light to be incident on the infrared light imaging means;
control means for determining appropriateness of the pattern portion printed on the sheet by using the ordinary ink, by means of the visible light from the white light source and the infrared light source which is reflected on the sheet to be incident on the two visible light imaging means and for determining appropriateness of the portion processed by using the mirror-surfaced member in the sheet, by means of the infrared light from the white light source and the infrared light source which is reflected on the sheet to be incident on the one infrared light imaging means.

2. The inspection device for a sheet according to claim 1, **characterized in that** the one infrared light imaging means is provided between the two visible light imaging means provided away from each other in a width direction of the sheet.

3. The inspection device for a sheet according to claim 1 or 2, **characterized in that** the inspection device further comprises an imaging means supporting member supporting all of the two visible light imaging means and the one infrared light imaging means.

4. The inspection device for a sheet according to any one of claims 1 to 3, **characterized in that** the two visible light imaging means and the one infrared light imaging means are provided at positions away from an imaged surface of the sheet by an equal distance.

5. The inspection device for a sheet according to any one of claims 1 to 4, **characterized in that** the inspection device further comprises a light source supporting member which supports both of the white light source and the infrared light source such that the light from the white light source and the light from the infrared light source fall on an imaged surface of the sheet imaged by the two visible light imaging means and the one infrared light imaging means and in which a supporting surface supporting the infrared light source is inclined with respect to a supporting surface supporting the white light source such that an angle formed by the imaged surface of the sheet and a direction of the light emitted by the infrared light source is smaller than an angle formed by the imaged surface of the sheet and a direction of the light emitted by the white light source.
